# EUROPEAN PATENT SPECIFICATION

(11) **EP 3 542 715 B1**
(45) Date of publication and mention of the grant of the patent: **11.05.2022**
(21) Application number: 18163325.6
(22) Date of filing: 22.03.2018
(51) Int. Cl.: A61B 5/024

(54) **DETECTOR ARRANGEMENT SUITED FOR OPTICAL SENSORS**
FÜR OPTISCHE SENSOREN GEEIGNETE DETEKTORANORDNUNG
AGENCEMENT DE DÉTECTEUR ADAPTÉ À DES CAPTEURS OPTIQUES

(43) Date of publication of application: 25.09.2019
(73) Proprietor: Nokia Technologies Oy, 02610 Espoo (FI)
(72) Inventor: BLOMQVIST, Kim, 02650 Espoo (FI); RUPONEN, Pellervo, 00530 Helsinki (FI)
(74) Representative: Espatent Oy

(56) References cited:
- EP-A1- 3 087 916
- WO-A1-2017/220806
- US-A1- 2017 325 729
- SUVRADIP GHOSH ET AL: "A fractal-based photodiode for on-chip energy harvesting", SENSORS, 2010 IEEE, IEEE, PISCATAWAY, NJ, USA, 1 November 2010 (2010-11-01), pages 1169-1172, XP031978119, DOI: 10.1109/ICSENS.2010.5690657 ISBN: 978-1-4244-8170-5

## Description

### TECHNICAL FIELD

The present application generally relates to a detector arrangement suited for optical sensors. Some example embodiments are suited for physiological measurement sensors.

### BACKGROUND

This section illustrates useful background information without admission of any technique described herein representative of the state of the art.

Various measurement devices that measure physiological parameters of a subject such as pulse sensors can be used for measuring heart rate, movements or other personal parameters. The measurements can be performed for example by using a sensor device in a chest strap that is worn under clothes or by using a wrist worn watch-like sensor device.

Heart rate can be monitored for example optically using a photoplethysmogram (PPG) sensor. Such optical heart rate measurement is sensitive to movements. Motion artifacts caused by movements may corrupt the pulsatile heart rate (HR) signal and may confuse the HR monitoring algorithms of the sensor. The end result is that the calculated HR in beats per minute (BPM) may be wrong.

However, keeping the sensor completely stably on the wrist is difficult in practice. For example, the wrist strap cannot be kept too tight, because it would be unpleasant for the user and might even stop or deteriorate blood circulation in small vessels thereby causing the measurement signal to disappear. Also movements of the subject wearing the sensor may cause motion artifacts. Therefore motion artifacts cannot be completely avoided.

In addition to PPG sensors, motion artifacts may exist also in other optical sensors used for physiological measurements.

US20170325729 discloses a detector structure with detectors that have interdigitated shape. EP3087916 and WO2017220806 disclose detector structures with two detectors side by side.

### SUMMARY

Various aspects of example embodiments are set out in the claims. The embodiments and features, if any, described in this specification that do not fall under the scope of the independent claim are to be interpreted as examples useful for understanding various embodiments of the invention.

According to the present invention, there is provided an apparatus according to claim 1, which comprises a photodetector area comprising a first photodetector area and a second photodetector area, the first and second photodetector areas having co-planar interlocking shapes, and an optical blocking filter configured to filter light incident on the second photodetector area.

The first photodetector area comprises one or more first subareas that extend from an edge of the photodetector area into the photodetector area, and the second photodetector area comprises one or more second subareas that extend from an edge of the photodetector area into the photodetector area, and at least one of the first subareas has a shape that interlocks with a shape of at least one of the second subareas.

At least one of the first subareas and/or at least one of the second subareas extends from the edge of the photodetector area more than halfway across the photodetector area, towards an opposite edge of the photodetector area.

In an example embodiment, at least one of the first subareas and/or at least one of the second subareas extends from one edge of the photodetector area to an opposite edge of the photodetector area.

Herein it is to be noted that the edges between which the first and second subareas extend may be the same edges of the photodetector area or different edges of the photodetector area. The first subarea may extend for example from a left edge to a right edge while the second subarea extends from a lower edge to an upper edge, or vice versa.

In an example embodiment, the photodetector area comprises one or more inactive subareas.

In an example embodiment, a fractal space-filling curve defines shapes of the first photodetector area and the second photodetector area.

In an example embodiment, the fractal space-filling curve is one of: a peano curve, a moore curve, and a hilbert curve. Also some other curve may be used.

In an example embodiment, the interlocking shapes of the first photodetector area and the second photodetector area have been defined by a genetic algorithm.

In an example embodiment, size of the first photodetector area and the size of the second photodetector area are substantially equal.

In an example embodiment, the apparatus is configured to detect a target wavelength, and said optical blocking filter is configured to block the target wavelength. In an example embodiment, the apparatus further comprises a light source configured to emit light at the target wavelength.

In an example embodiment, the apparatus comprises an angle adjustment element configured to adjust the angle of incidence of light incident on the photodetector area.

In an example embodiment, the apparatus comprises analog circuitry configured to subtract an output signal provided by the second photodetector area from an output signal provided by the first photodetector area.

In an example embodiment, the apparatus comprises a signal processing element configured to produce a physiological measurement result using at least one of: an output signal provided by the first photodetector area and an output signal provided by the second photodetector area.

In an example embodiment, the apparatus comprises a mirror arranged to reflect light back to the first and/or second photodetector areas.

In an example embodiment, the apparatus is a physiological measurement sensor.

According to a second example aspect, there is provided a user wearable apparatus comprising any apparatus defined in the foregoing.

Different non-binding example aspects and embodiments have been illustrated in the foregoing. The embodiments in the foregoing are used merely to explain selected aspects or steps that may be utilized in implementations according to the present disclosure. Some embodiments may be presented only with reference to certain example aspects. It should be appreciated that corresponding embodiments may apply to other example aspects as well.

### BRIEF DESCRIPTION OF THE DRAWINGS

For a more complete understanding of example embodiments of the present disclosure, reference is now made to the following descriptions taken in connection with the accompanying drawings in which:
Fig. 1 is a simplified illustration of an example optical heart rate measurement;
Fig. 2 is a simplified illustration of an example user wearable sensor device;
Fig. 3 is a cross sectional view of a sensor device of an example embodiment;
Figs. 4A-5C show top views of detector structures;
Fig. 6 is a graph illustrating sensitivity of some example embodiments;
Fig. 7 is a flow chart of a process of an example embodiment, and
Fig. 8 is a flow chart of a process of another example embodiment.

### DETAILED DESCRIPTON OF THE DRAWINGS

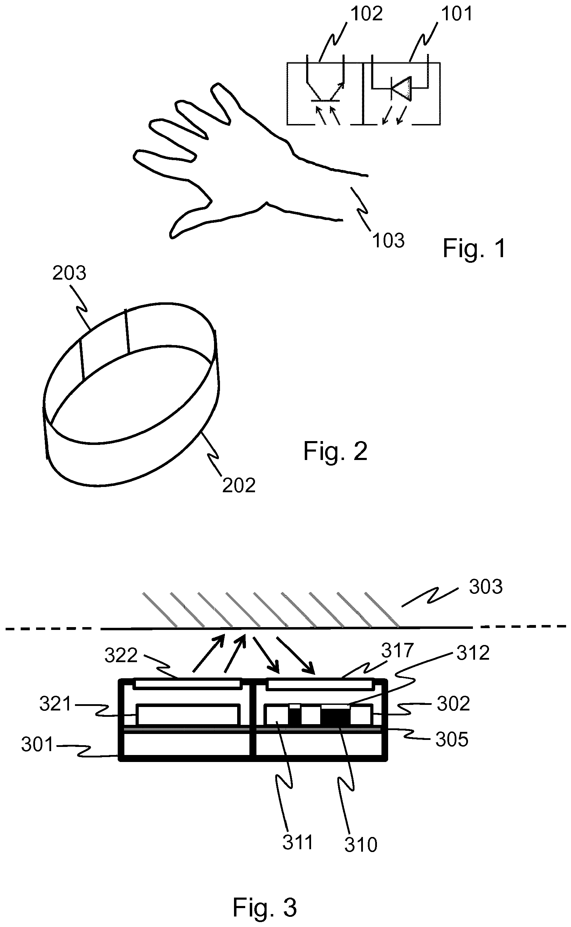

Example embodiments of the present disclosure and its potential advantages are understood by referring to Figs. 1 through 8 of the drawings. In this document, like reference signs denote like parts or steps.

In various example embodiments there is provided a new detector arrangement suited for being used in optical sensors for example for measuring physiological parameters of a subject. Such optical sensors measure physiological parameters of the subject and produce sensor signals corresponding to a property of the matter underlying the skin of the subject (capillaries and veins, for example). The detector arrangement is suited e.g. for user wearable sensor devices.

In the following, various example embodiments are discussed in connection with optical heart rate sensors. Various example embodiments are however not necessarily limited to optical heart rate sensors only. For example, alternatively or additionally the example embodiments can be used in optical monitoring of some other physiological parameter, too. Physiological parameters or physiological measurement results referred to herein may include for example one or more of the following: heart rate, respiration rate, blood pressure, blood oxygen saturation level, and blood glucose level.

Heart rate can be monitored optically by measuring variations in blood volume with a photoplethysmogram (PPG) sensor. Fig. 1 is a simplified illustration of an example optical heart rate sensor. Fig. 1 shows a (reflective type) PPG sensor that comprises a light source 101 and a photodetector 102. The light source in the shown example is a LED (light emitting diode) and the photodetector is a phototransistor. Also a photo diode (PD) may be used. The light source 101 emits light and the photodetector 102 receives light rays reflected from tissue 103 of a subject. The sensor produces sensor signals based on the light detected by the photodetector 102.

In an example embodiment there is provided an apparatus (e.g. an optical sensor) with a photodetector area comprising a first photodetector area and a second photodetector area. The first and second photodetector areas have co-planar interlocking shapes. The first and second photodetector areas are configured to concurrently detect incident light. In an example embodiment a first photodetector area is configured to receive a range of wavelengths and a second photodetector area is configured not to receive a target wavelength. In an example embodiment, the target wavelength is a wavelength of interest. The target wavelength may be indicated in terms of a single wavelength, for example 525 nm, but it is understood that the target wavelength may be a narrow wavelength band around the stated wavelength.

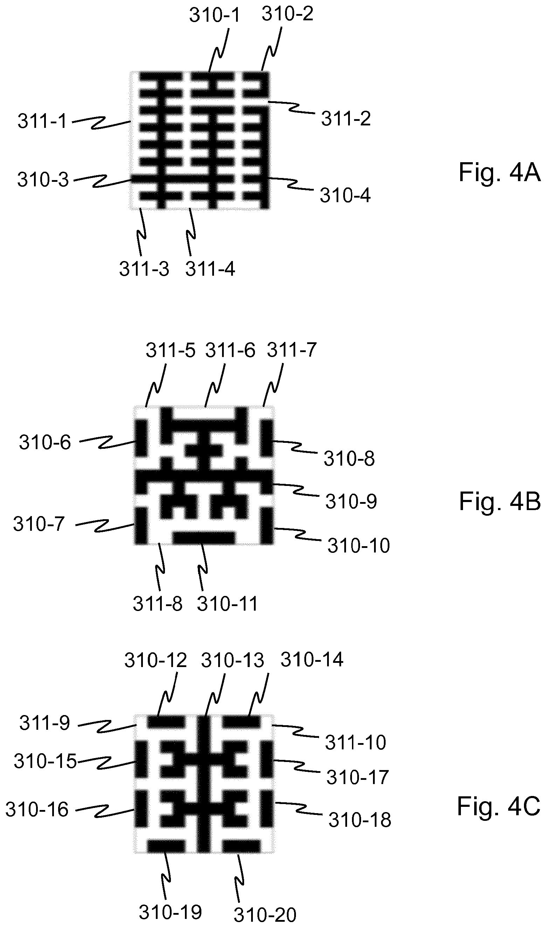

The phrase "co-planar interlocking shapes" refers to shapes that interlock with each other so that the shape of the first photodetector area limits movements of the second photodetector area in at least two directions in a two-dimensional co-planar plane. For example, squares of a checker pattern do not interlock with each other as they may limit the movement of other shapes only in one direction in a two-dimensional plane. It is noted that co-planar shapes are nonoverlapping. They may reside side by side, but they do not overlap each other. The co-planar interlocking shapes may be defined by a genetic algorithm or by fractal space-filling curves. More detailed examples of these are provided in connection with Figs. 4A-5C.

The first photodetector area is formed of a plurality of first subareas and the second photodetector area is formed of a plurality of second subareas. The signals detected by the subareas may be processed so that signals detected by the first subareas are combined and signals detected by second subareas are combined. At least one of the first subareas is such that it interlocks with at least one of the second subareas. Additionally there may be first and/or second subareas that do not have an interlocking shape. Still further, the photodetector area may comprise inactive subareas, i.e. areas that are not configured to detect light.

The photodetector area may be of any suitable size. The photodetector area may be divided into a plurality of pixels and the photodetector area may cover for example an area of 11x11 pixels or alternatively an area of 5x5, 9x9 or 13x13 pixels. Also other sizes are possible. In an example embodiment, sizes of the first photodetector area and the second photodetector area are substantially equal. This means that the first photodetector area and the second photodetector area cover the same or at least almost the same amount of pixels of the photodetector area. For example in an 11x11 pixel area (121 pixels in total), the first and second photodetector areas may both cover 60 pixels, while one pixel is inactive. In another example, the first photodetector area covers 61 pixels and the second photodetector area covers 59 pixels and one pixel is inactive. In yet another example, the first photodetector area covers 59 pixels and the second photodetector area covers 62 pixels or vice versa. In still another example, the first photodetector area covers 61 pixels and the second photodetector area covers 60 pixels or vice versa.

The inactive pixel (or pixels) may make the arrangement less sensitive to angle of incidence of light in some cases. Assigning certain pixel to either the first photodetector area or the second photodetector area may not be beneficial in some cases. In such cases, inactive pixel or pixels may be used to reduce effects of angular dependency of the response of the first and second photodetector areas. For example, it may be desirable to have equal number of pixels for both the first photodetector area and the second photodetector area but the total number of pixels in the photodetector area may be uneven. In such cases making one pixel inactive allows one to have equal number of pixels for both the first photodetector area and the second photodetector area. In many example embodiments there is one inactive pixel, but also more than one inactive pixels may be used.

The shapes of the first photodetector area and the second photodetector area may form a symmetrical pattern on the photodetector area, but this is not a mandatory feature and also asymmetrical patterns may be used.

According to the invention, each first and second subarea extends from an edge of the photodetector area into the photodetector area in a two-dimensional coplanar plane. At least one of the first or second subareas extends from the edge of the photodetector area more than halfway across the photodetector area, towards an opposite edge of the photodetector area. In an example embodiment, at least one of the first or second subareas extends from one edge of the photodetector area to an opposite edge of the photodetector area. Herein it is to be noted that the edges between which the first and second subareas extend may be the same edges of the photodetector area or different edges of the photodetector area. The first subarea may extend for example from a left edge to a right edge while the second subarea extends from a lower edge to an upper edge, or vice versa. Alternatively, the first subarea and the second subarea may extend for example from a left edge to a right edge or from a lower edge to an upper edge. Additionally or alternatively, there may be a plurality of first subareas and/or second subareas that extend from one edge to another edge.

According to the invention, the apparatus comprises an optical blocking filter that is configured to filter the light incident at the second photodetector area. The optical blocking filter may be placed in a stacked arrangement with the second photodetector area. The optical blocking filter and the second photodetector area may be in contact with each other or there may be a space between them. The optical blocking filter blocks some wavelengths. The optical blocking filter may be for example a notch filter or a band-stop filter, and may comprise a dichroic mirror. Filters that use dichroic mirrors can be very steep and therefore they may suit well for example embodiments of present disclosure because they enable precise separation of wavelengths. In an example embodiment the optical blocking filter is configured to block a target wavelength, which is the wavelength of interest. In an ideal implementation the optical blocking filter blocks 100% of the target wavelength. It is however understood that in practical implementations a minimal amount of the target wavelength may leak through the optical blocking filter. It is understood that blocking covers also situations where such leakage may exist. In this way, the second photodetector area does not receive the target wavelength. In an example embodiment the target wavelength is 525 nm wavelength (green light), 650 nm wavelength (red light) or 950 nm wavelength (infrared light), but other wavelengths can be equally used.

In an example embodiment, the apparatus comprises an angle adjustment element that is configured to adjust the angle of light incident on the apparatus. In general the angle adjustment element is an element that affects the angle of light that passes through the element. The angle adjustment element may be an angle-limiting filter that is configured to block light rays with undesired angle of incidence or it may be a collimator configured to change direction of incident light rays so that the light rays exiting or passing through the collimator have a desired angle. It is to be understood that the angle adjustment element is not a mandatory element even though an angle adjustment element is included by way of example in many of the example embodiments discussed herein.

In an example embodiment, the apparatus comprises a light source that emits light at a wavelength that may be the target wavelength. The optical blocking filter matches the wavelength of the light source. That is, the optical blocking filter is configured to filter out the wavelength of the light source.

Now, when the first and second photodetector areas detect light, the second photodetector area (which does not receive the target wavelength) can be considered to detect only unwanted wavelengths. The detected light signals may be subtracted from each other to produce a result signal that is cleared from noise and artifacts originating from the unwanted wavelengths. In an example embodiment there is provided an analog circuit configured to perform the subtraction. In another alternative the detected signals are analog-to-digital converted and then subtracted digitally. The resulting signal may then be used for producing a physiological measurement result, such as heart rate. Additionally, the signals detected by the first and second photodetector areas may be used as such, i.e. without being subtracted from each other, for producing physiological measurement results.

Fig. 2 is a simplified illustration of an example sensor 203 wherein example embodiments may be implemented. The sensor 203 is attached to a strap 202 that allows the sensor 203 to be fitted for example around a wrist of a subject.

The casing of the sensor 203 can be made of a suitable material, such as a plastics material (e.g. acrylonitrile butadiene styrene (ABS) or polycarbonate (PC)), carbon fiber materials, glass, wood, metal, ceramics, fabric, leather or any combination of these. The strap 202 may be made of suitable flexible material, such as plastic, fabric, and leather. In an example embodiment, the strap 202 and the casing of the sensor 203 are integrally formed of one piece of material. The material can comprise or consist for example of any of the following: plastics, metals, nano-fibers, carbon fiber, leather, fabric and glass.

Fig 2 shows the sensor 203 attached to a strap 202, but the sensor may equally be part of some other user wearable apparatus that can be fitted around or attached to a body part (e.g. wrist, ankle or finger) of a user. The sensor 203 may be configured to be integrated into a garment of a subject. The sensor may be attached or integrated for example to a belt, a sock, a shoe, a sleeve or a collar of a shirt or pullover, and/or a waistband of trousers or skirt. The sensor may be detachable from the garment. The sensor may be shaped like a watch and it may be configured to display time or other useful information to the user. The sensor may be attached to a patch or to a plaster (with adhesive) or to a ring. A further alternative is that the sensor is attached to an ear of the user. The sensor may be part of an earplug, for example.

Fig. 3 is a cross sectional view of a sensor device of an example embodiment. Fig. 3 shows tissue 303 of a subject and a sensor device 301 comprising a printed circuit board (PCB) 305, a photodetector area 302, and a light source 321. Further there is a window element 322 that allows light emitted by the light source 321 to exit the apparatus 301 and a window element 317 that allows light to be received by the apparatus 301. It is to be noted that the window elements 322 and 317 are not mandatory. Instead the apparatus 301 may comprise apertures that allow light to exit and enter the apparatus 301. In an embodiment the window element 317 comprises an angle adjustment element that is configured to adjust the angle of light incident on the apparatus 301.

The photodetector area 302 comprises first photodetector areas 311, second photodetector areas 310, and an optical blocking filter 312 arranged to filter light incident on the second photodetector area 310.

In an example embodiment, the apparatus 301 of Fig 3 operates as follows: The light source 321 emits light through the window element 322. The light is reflected from the tissue 303 of the subject and the light enters the photodetector area 302 through the window element 317. The first photodetector area 311 and the second photodetector area 310 receive light having substantially the same angle of incidence. Additionally, the first photodetector area 311 and the second photodetector area 310 are configured to receive substantially the same amount of incident light. The terms substantially same angle and substantially same amount of light refer to the same or almost same angle or amount. That is, the angle of incidence and the amount of light may be slight different.

The first photodetector area 311 detects all wavelengths of the light incident on the photodetector area 302. The second photodetector area 310 detects all other wavelengths of the light incident on the photodetector area 302 except the wavelength blocked by the optical blocking filter 312. The first photodetector area 311 produces a first detected signal and the second photodetector area 310 produces a second detected signal. These detected signals or either one of them may then be used for providing physiological measurement results. There may be for example a signal processing element (not shown in Fig. 3) configured to produce the physiological measurement results. In an embodiment there is an analog circuitry configured to subtract an output signal provided by the second photodetector area from an output signal provided by the first photodetector area. The signal processing element may comprise a processor such as a microprocessor or a digital signal processor. Additionally the signal processing element may comprise one or more analog components such as amplifiers and impedances and/or analog-digital converters.

The first photodetector area is formed of one or more first subareas and the second photodetector area is formed of one or more second subareas. The signals detected by the subareas may be processed so that signals detected by the first subareas are combined and signals detected by second subareas are combined. The resulting signals may then be subtracted or used as such for producing the physiological measurement results.

In yet another example embodiment the sensor device of Fig. 3 comprises a mirror arranged to reflect light back to the first and/or second photodetector areas. Such mirror may increase the amount of light detected by the photodetector areas. A portion of light incident on the first and second photodetector areas 310 and 311 may leak through the first and second photodetector areas 310 and 311. The mirror is provided in a suitable location such that it reflects this light back to the first and/or second photodetector areas 310, 311 and thereby increases the amount of light the first and second photodetector areas 310, 311 detect. The mirror may be placed between the PCB 305 and the photodetector area 302.

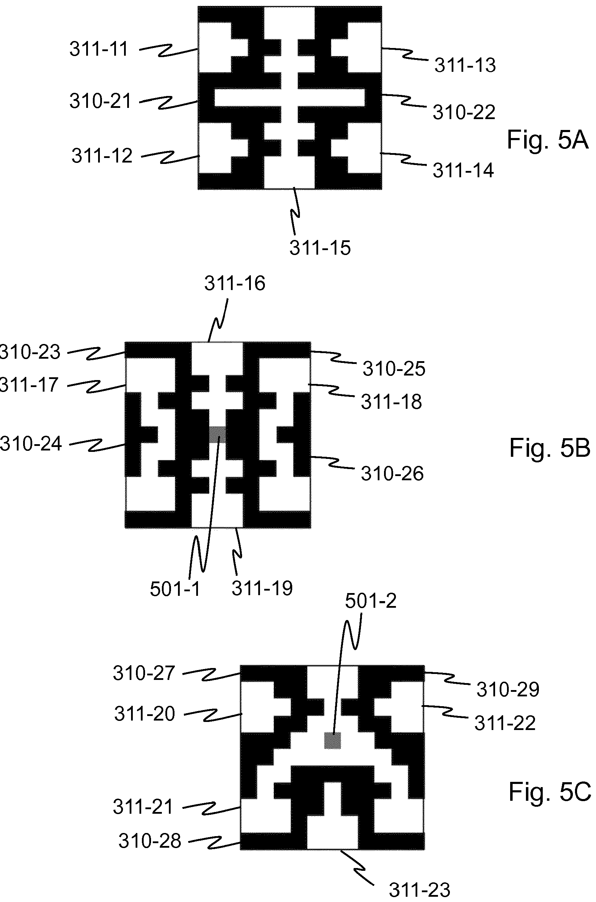

Figs. 4A-5C are top views of detector structures. In the detector structures of Figs. 4A-4C a fractal space-filling curve defines shapes of a first photodetector area and a second photodetector area. The detector structures of Figs. 5A-5C comprise a first photodetector area and a second photodetector area having interlocking shapes that have been defined by a genetic algorithm.

A space-filling curve is a curve whose range contains the entire 2-dimensional unit square (or more generally an n-dimensional unit hypercube). Various space-filling curves are known and this disclosure is not limited to any specific curve. Some examples of suitable space-filling curves are shown in Figs. 4A-4C, but clearly also other fractal space-filling curves may be employed.

Fig 4A shows a detector structure, wherein a Peano curve divides a photodetector area into a first photodetector area and a second photodetector area. The first photodetector area is formed of first subareas 311-1 to 311-4. The second photodetector area is formed of second subareas 310-1 to 310-4.

Fig 4B shows a detector structure, wherein a Hilbert curve divides a photodetector area into a first photodetector area and a second photodetector area. The first photodetector area is formed of first subareas 311-5 to 311-8. The second photodetector area is formed of second subareas 310-6 to 310-11.

Fig 4C shows a detector structure, wherein a Moore curve divides a photodetector area into a first photodetector area and a second photodetector area. The first photodetector area is formed of first subareas 311-9 to 311-10. The second photodetector area is formed of second subareas 310-12 to 310-20.

It is to be noted that examples embodiments of Figs. 4A-4C do not show any inactive pixels. Nevertheless, alternative example embodiments comprising shapes defined by space-filling curves may comprise also inactive pixels.

In computer science, a genetic algorithm is a metaheuristic inspired by natural selection. In a genetic algorithm, a population of candidate solutions to an optimization problem is iteratively evolved toward better solutions.

According to an example definition, a genetic algorithm operates as follows: The evolution usually starts from a population of randomly generated individuals, and is an iterative process, with the population in each iteration called a generation. In each generation, the fitness of every individual in the population is evaluated; the fitness is usually the value of the objective function in the optimization problem being solved. The more fit individuals are stochastically selected from the current population, and each individual's genome is modified (recombined and possibly randomly mutated) to form a new generation. The new generation of candidate solutions is then used in the next iteration of the algorithm. Commonly, the algorithm terminates when either a maximum number of generations has been produced, or a satisfactory fitness level has been reached for the population.

Some examples of photodetector patterns developed using a genetic algorithm are shown in Figs. 5A-5C, but clearly also other patterns are possible and the present disclosure is not limited only to these example patterns.

Fig 5A shows a detector structure, wherein a first photodetector area is formed of first subareas 311-11 to 311-15 and a second photodetector area is formed of second subareas 310-21 to 310-22.

Fig 5B shows a detector structure, wherein a first photodetector area is formed of first subareas 311-17 to 311-19 and a second photodetector area is formed of second subareas 310-23 to 310-26. Additionally the pattern of Fig 5B comprises an inactive area 501-1.

Fig 5B shows a detector structure, wherein a first photodetector area is formed of first subareas 311-20 to 311-23 and a second photodetector area is formed of second subareas 310-27 to 310-29. Additionally the pattern of Fig 5B comprises an inactive area 501-2.

It is to be noted that in the examples of Figs. 4A-5C, each first and second subarea extends to an edge of the photodetector area. In this way it is easier to provide contact to the subareas.

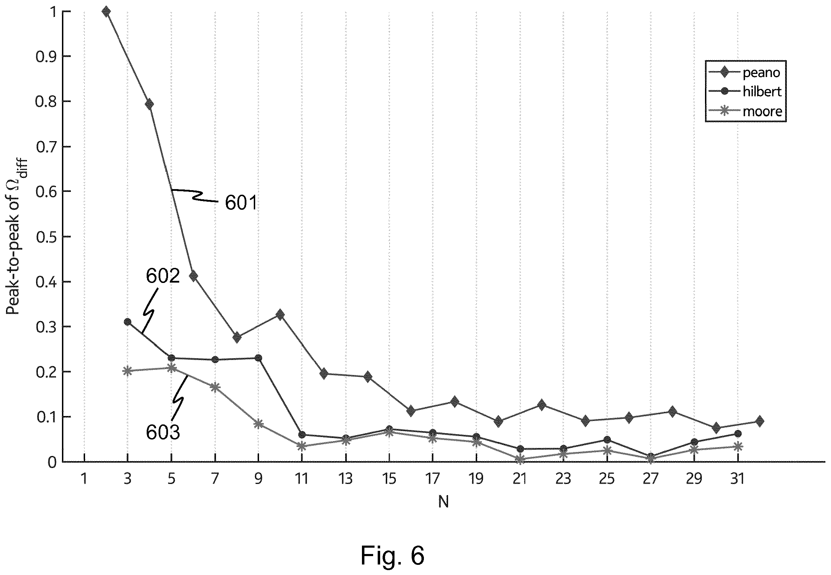

Fig. 6 is a graph illustrating sensitivity of detector patterns of some example embodiments. Values on curve 601 show sensitivity of pattern structures that comprise a Peano curve shape, values on curve 602 show sensitivity of pattern structures that comprise a Hilbert curve shape, and values on curve 603 show sensitivity of pattern structures that comprising a Moore curve shape.

X-axis represents detector resolution in pixels (the actual detector area size in millimeters staying the same) and Y-axis represents sensitivity of the pattern to angle of light beam incident on the detector area. From the graph it can be seen that with detector size N=11 the results of different patterns start to converge and further increase in resolution does not provide significant improvement in sensitivity. Thereby one may choose to use detectors with 11x11 pixels. Nevertheless, other resolutions can be used, too.

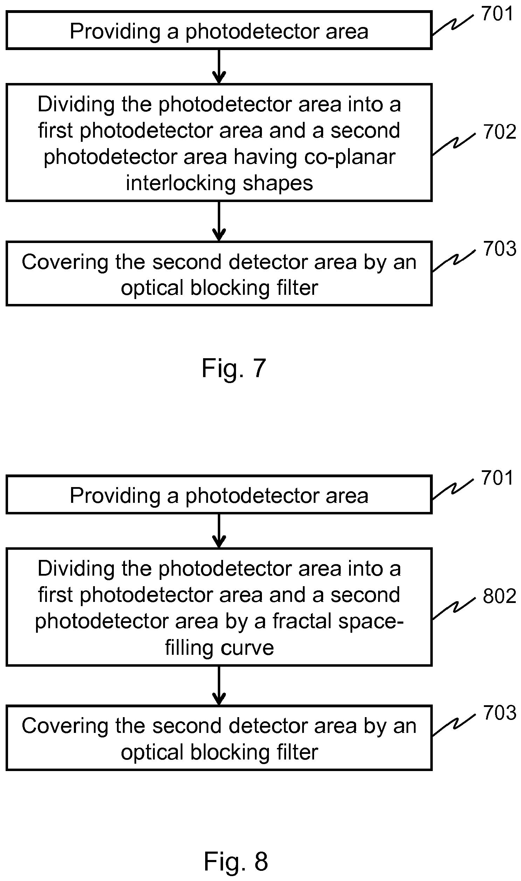

Fig. 7 shows a flow chart of a process of an example embodiment. The process comprises:
701: Photodetector area is provided. The area may have any suitable size, such as e.g. 1 mm² and any suitable resolution, such as 11x11 pixels.
702: The photodetector area is divided into a first photodetector area and a second photodetector area having co-planar interlocking shapes. The first and second photodetector areas cover the whole photodetector area or there may be one or more inactive areas.
703: The second detector area is covered by an optical blocking filter.

Fig. 8 shows a flow chart of a process of an example embodiment. The process comprises:
701: Photodetector area is provided. The area may have any suitable size, such as e.g. 1 mm² and any suitable resolution, such as 11x11 pixels.
802: The photodetector area is divided into a first photodetector area and a second photodetector area having co-planar interlocking shapes. The division into the first and second photodetector areas is performed by defining shapes of the first photodetector area and second photodetector area by a fractal space-filling curve.
703: The second detector area is covered by an optical blocking filter.

Without in any way limiting the scope, interpretation, or application of the claims appearing below, a technical effect of one or more of the example embodiments disclosed herein is that an improved optical detector arrangement is provided. The detector arrangement of example embodiments provides that the first photodetector area and the second photodetector area detect light with substantially the same angle of incidence. This eliminates or at least reduces effects of angular dependency of the response of the first and second photodetector areas. In this way the first and second photodetector areas produce comparable detected signals. Thus, accuracy of noise cancellation that uses signals from both the first and the second photodetector areas may be improved. Thereby, accuracy of overall measurement may be improved.

Another technical effect of one or more of the example embodiments disclosed herein is that analog noise cancellation is enabled. Analog noise cancellation may be faster and more robust than digital solutions. A benefit of analog noise cancellation is that it is directed to removing or reducing the component causing inaccuracies in measurement signal (i.e. removing the original cause).

Another technical effect of one or more of the example embodiments disclosed herein is that DC component of a measured signal is reduced. In heart rate monitoring applications DC is not an interesting component and the presence of the DC component only narrows down the effective dynamic range of an analog front- end.

Another technical effect of one or more of the example embodiments disclosed herein is that the solution is easy to take into use. Various example solutions are compatible with existing optical measurement ICs (integrated circuits) and/or can be easily made compatible with existing optical measurement ICs. Additionally the shapes of the photodetector areas are feasible to be implemented in standard semiconductor device fabrication and thus may be cheap to manufacture.

Another technical effect of one or more of the example embodiments disclosed herein is that the solution is less sensitive to ambient and other unwanted light sources. Another technical effect of one or more of the example embodiments disclosed herein is that a wide spectrum light source can be used. For example wider spectrum than spectrum provided by LEDs could be used.

If desired, the different functions discussed herein may be performed in a different order and/or concurrently with each other. It is understood that desired features from one explicitly disclosed example embodiment may be combined with a selection of features from other example embodiments.

Although various aspects of present disclosure are set out in the independent claims, other aspects of the present disclosure comprise other combinations of features from the described example embodiments and/or the dependent claims with the features of the independent claims, and not solely the combinations explicitly set out in the claims.

It is also noted herein that while the foregoing describes example embodiments, these descriptions should not be viewed in a limiting sense. The example embodiments are not limited to the examples explicitly described in this specification but may vary within the scope of the appended claims.

## Claims

1. An apparatus comprising:
a photodetector area (302) comprising a first photodetector area (311, 311-1 - 311-22) and a second photodetector area (310, 310-1 - 310-29), and
an optical blocking filter (312) configured to filter light incident on the second photodetector area, wherein the first and second photodetector areas have co-planar interlocking shapes, so that the shape of the first photodetector area limits movements of the second photodetector area in at least two directions in a two-dimensional co-planar plane,
the first photodetector area comprises a plurality of first subareas (311-1 - 311-22) that extend from an edge of the photodetector area into the photodetector area, and
the second photodetector area comprises a plurality of second subareas (310-1 - 310-29) that extend from an edge of the photodetector area into the photodetector area, wherein
at least one of the first subareas has a shape that interlocks with a shape of at least one second subarea, and
at least one of the first subareas and/or at least one of the second subareas extends from the edge of the photodetector area (302) more than halfway across the photodetector area, towards an opposite edge of the photodetector area (302).

2. The apparatus of claim 1, wherein at least one of the first subareas and/or at least one of the second subareas extends from one edge of the photodetector area (302) to an opposite edge of the photodetector area (302).

3. The apparatus of any preceding claim, wherein the photodetector area (302) comprises one or more inactive subareas (501-1, 501-2).

4. The apparatus of any preceding claim, wherein a fractal space-filling curve defines shapes of the first photodetector area (311, 311-1 - 311-10) and the second photodetector area (310, 310-1 - 310-20).

5. The apparatus of claim 4, wherein said fractal space-filling curve is one of: a peano curve, a moore curve, and a hilbert curve.

6. The apparatus of any one of claims 1-3, wherein the interlocking shapes of the first photodetector area (311, 311-11 - 311-22) and the second photodetector area (310, 310-21 - 310-29) have been defined by a genetic algorithm.

7. The apparatus of any preceding claim, wherein size of the first photodetector area (311, 311-1 - 311-22) and the size of the second photodetector area (310, 310-1 - 310-29) are substantially equal.

8. The apparatus of any preceding claim, wherein
the apparatus is configured to detect a target wavelength, and
said optical blocking filter (312) is configured to block the target wavelength.

9. The apparatus of claim 8, further comprising
a light source (321) configured to emit light at the target wavelength..

10. The apparatus of any preceding claim, further comprising an angle adjustment element configured to adjust the angle of incidence of light incident on the photodetector area.

11. The apparatus of any preceding claim, further comprising a signal processing element configured to produce a physiological measurement result using at least one of: an output signal provided by the first photodetector area (311, 311-11 - 311-22) and an output signal provided by the second photodetector area (310, 310-1 - 310-29).

12. The apparatus of any preceding claim, further comprising a mirror arranged to reflect light back to the first and/or second photodetector areas.

13. The apparatus of any preceding claim, wherein the apparatus is a physiological measurement sensor.

## Patentansprüche

1. Vorrichtung, die Folgendes umfasst:
einen Photodetektorbereich (302), der einen ersten Photodetektorbereich (311, 311-1 - 311-22) und einen zweiten Photodetektorbereich (310, 310-1 - 310-29) umfasst, und
ein optisches Sperrfilter (312), das dazu ausgelegt ist, Licht, das in den zweiten Photodetektorbereich einfällt, zu filtern, wobei der erste und der zweite Photodetektorbereich koplanare ineinandergreifende Formen aufweisen, derart, dass die Form des ersten Photodetektorbereichs Bewegungen des zweiten Photodetektorbereichs in mindestens zwei Richtungen in einer zweidimensionalen koplanaren Ebene beschränkt,
der erste Photodetektorbereich umfasst eine Vielzahl von ersten Unterbereichen (311-1 - 311-22), die sich von einem Rand des Photodetektorbereichs in den Photodetektorbereich erstrecken, und
der zweite Photodetektorbereich umfasst eine Vielzahl von zweiten Unterbereichen (310-1 - 310-29), die sich von einem Rand des Photodetektorbereichs in den Photodetektorbereich erstrecken, wobei
mindestens einer der ersten Unterbereiche eine Form aufweist, die in eine Form von mindestens einem zweiten Unterbereich eingreift, und
mindestens einer der ersten Unterbereiche und/oder mindestens einer der zweiten Unterbereiche sich vom Rand des Photodetektorbereichs (302) um mehr als die Hälfte über den Photodetektorbereich zu einem gegenüberliegenden Rand des Photodetektorbereichs (302) erstreckt.

2. Vorrichtung nach Anspruch 1, wobei sich mindestens einer der ersten Unterbereiche und/oder mindestens einer der zweiten Unterbereiche von einem Rand des Photodetektorbereichs (302) zu einem gegenüberliegenden Rand des Photodetektorbereichs (302) erstreckt.

3. Vorrichtung nach einem der vorhergehenden Ansprüche, wobei der Photodetektorbereich (302) einen oder mehrere inaktive Unterbereiche (501-1, 501-2) umfasst.

4. Vorrichtung nach einem der vorhergehenden Ansprüche, wobei eine fraktale raumfüllende Kurve Formen des ersten Photodetektorbereichs (311, 311-1 - 311-10) und des zweiten Photodetektorbereichs (310, 310-1 - 310-20) definiert.

5. Vorrichtung nach Anspruch 4, wobei die fraktale raumfüllende Kurve eines von Folgendem ist: eine Peano-Kurve, eine Moore-Kurve und eine Hilbert-Kurve.

6. Vorrichtung nach einem der Ansprüche 1-3, wobei die ineinandergreifenden Formen des ersten Photodetektorbereichs (311, 311-11 - 311-22) und des zweiten Photodetektorbereichs (310, 310-21 - 310-29) von einem genetischen Algorithmus definiert wurden.

7. Vorrichtung nach einem der vorhergehenden Ansprüche, wobei eine Größe des ersten Photodetektorbereichs (311, 311-1 - 311-22) und die Größe des zweiten Photodetektorbereichs (310, 310-1 - 310-29) im Wesentlichen gleich sind.

8. Vorrichtung nach einem der vorhergehenden Ansprüche, wobei
die Vorrichtung dazu ausgelegt ist, eine Zielwellenlänge zu detektieren, und
das optische Sperrfilter (312) dazu ausgelegt ist, die Zielwellenlänge zu sperren.

9. Vorrichtung nach Anspruch 8, die ferner Folgendes umfasst
eine Lichtquelle (321), die dazu ausgelegt ist, Licht mit der Zielwellenlänge zu emittieren.

10. Vorrichtung nach einem der vorhergehenden Ansprüche, die ferner ein Winkelanpassungselement umfasst, das dazu ausgelegt ist, den Einfallswinkel von Licht, das in den Photodetektorbereich einfällt, anzupassen.

11. Vorrichtung nach einem der vorhergehenden Ansprüche, die ferner ein Signalverarbeitungselement umfasst, das dazu ausgelegt ist, unter Verwendung von mindestens einem von Folgendem ein physiologisches Messergebnis zu produzieren: ein Ausgangssignal, das vom ersten Photodetektorbereich (311, 311-11 - 311-22) bereitgestellt wird, und ein Ausgangssignal, das vom zweiten Photodetektorbereich (310, 310-1 - 310-29) bereitgestellt wird.

12. Vorrichtung nach einem der vorhergehenden Ansprüche, die ferner einen Spiegel umfasst, der angeordnet ist, Licht zum ersten und/oder zum zweiten Photodetektorbereich zurück zu reflektieren.

13. Vorrichtung nach einem der vorhergehenden Ansprüche, wobei die Vorrichtung ein physiologischer Messsensor ist.

## Revendications

1. Appareil qui comprend :
une zone de photodétecteur (302) qui comprend une première zone de photodétecteur (311, 311-1 - 311-22) et une seconde zone de photodétecteur (310, 310-1 - 310-29), et
un filtre de blocage optique (312) configuré pour filtrer la lumière incidente sur la seconde zone de photodétecteur, dans lequel la première et la seconde zones de photodétecteurs possèdent des formes d'emboîtement coplanaires, de sorte que la forme de la première zone de photodétecteur limite les mouvements de la seconde zone de photodétecteur dans au moins deux directions sur un plan coplanaire en deux dimensions,
la première zone de photodétecteur comprend une pluralité de premières sous-zones (311-1 - 311-22) qui s'étendent depuis un bord de la zone de photodétecteur dans la zone de photodétecteur, et
la seconde zone de photodétecteur comprend une pluralité de secondes sous-zones (310-1 - 310-29) qui s'étendent depuis un bord de la zone de photodétecteur dans la zone de photodétecteur, dans lequel
au moins l'une des premières sous-zones possède une forme qui s'emboîte avec une forme d'au moins une seconde sous-zone, et
au moins l'une des premières sous-zones et/ou au moins l'une des secondes sous-zones s'étend depuis le bord de la zone de photodétecteur (302) plus qu'à mi-chemin au sein de la zone de photodétecteur, vers un bord opposé de la zone de photodétecteur (302).

2. Appareil selon la revendication 1, dans lequel au moins l'une des premières sous-zones et/ou au moins l'une des secondes sous-zones s'étend depuis un bord de la zone de photodétecteur (302) vers un bord opposé de la zone de photodétecteur (302).

3. Appareil selon l'une quelconque des revendications précédentes, dans lequel la zone de photodétecteur (302) comprend une ou plusieurs sous-zone(s) inactive(s) (501-1, 501-2) .

4. Appareil selon l'une quelconque des revendications précédentes, dans lequel une courbe de remplissage d'espace fractal définit la forme de la première zone de photodétecteur (311, 311-1 - 311-10) et de la seconde zone de photodétecteur (310, 310-1 - 310-20).

5. Appareil selon la revendication 4, dans lequel ladite courbe de remplissage d'espace fractal est l'une d'une courbe de Peano, d'une courbe de Moore, et d'une courbe de Hilbert.

6. Appareil selon l'une quelconque des revendications 1 à 3, dans lequel les formes d'emboîtement de la première zone de photodétecteur (311, 311-11 - 311-22) et la seconde zone de photodétecteur (310, 310-21 - 310-29) ont été définies par un algorithme génétique.

7. Appareil selon l'une quelconque des revendications précédentes, dans lequel la taille de la première zone de photodétecteur (311, 311-1 - 311-22) et la taille de la seconde zone de photodétecteur (310, 310-1 - 310-29) sont sensiblement identiques.

8. Appareil selon l'une quelconque des revendications précédentes, dans lequel
l'appareil est configuré pour détecter une longueur d'onde cible, et
ledit filtre de blocage optique (312) est configuré pour bloquer la longueur d'onde cible.

9. Appareil selon la revendication 8, qui comprend en outre
une source de lumière (321) configurée pour émettre de la lumière à la longueur d'onde cible.

10. Appareil selon l'une quelconque des revendications précédentes, qui comprend en outre un élément de réglage d'angle configuré pour régler l'angle d'incidence de la lumière incidente sur la zone de photodétecteur.

11. Appareil selon l'une quelconque des revendications précédentes, qui comprend en outre un élément de traitement de signal configuré pour produire un résultat de mesure physiologique en utilisant au moins l'un de : un signal de sortie fourni par la première zone de photodétecteur (311, 311-11 - 311-22) et un signal de sortie fourni par la seconde zone de photodétecteur (310, 310-1 - 310-29).

12. Appareil selon l'une quelconque des revendications précédentes, qui comprend en outre un miroir prévu pour réfléchir la lumière vers la première et/ou la seconde zone de photodétecteur.

13. Appareil selon l'une quelconque des revendications précédentes, dans lequel l'appareil est un capteur de mesure physiologique.
